# EUROPEAN PATENT APPLICATION

(11) **EP 1 830 191 A2**
(43) Date of publication of application: **05.09.2007**
(21) Application number: 07109545.9
(22) Date of filing: 02.03.2004
(51) Int. Cl.: G01N 33/68

(54) **Pharmaceutical preparations containing thiazolidinediones showing new therapeutic indications**

(30) Priority: 06.03.2003 IT FI20030058
(62) Divisional of application: 04716242.5
(71) Applicant: Universita' Degli Studi di Firenze, 50121 Firenze (IT)
(72) Inventor: Galli, Andrea, 50144, FIRENZE (IT); Romagnani, Paola, 50139, FIRENZE (IT); Serio, Mario, 50012, BAGNO A RIPOLI (IT); Surrenti, Calogero, 50012, BAGNO A RIPOLI (IT)
(74) Representative: Gervasi, Gemma

(57) **Abstract**

The use of thiazolidinediones for the preparation of phannaceutical compositions useful for the treatment of endocrine autoimnune diseases is described

## Description

### Field of the invention

The present invention is connected to new therapeutic applications of thiazolidinediones also called glitazones, later on indicated as TzDs

### State of art

The TzDs represent a class of drug recently approved to improve glycemic control in patients affected by type 2 diabetes mellitus. The first of this class of drug, the ciglitazone, has been synthesized in 1982 (Fujita al 1983) and in spite of the study of this molecule has been interrupted, several compounds have been developed with different pharmacokinetics characteristics as Rosiglitazone and Pioglitazone, which are now commercially available.

A large body of experimental data seems to demonstrate that the majority of TzDs effects are due to interaction with PPARγ.

The hypoglicemic effect of TzDs is due to the improvement of the peripherical action of insulin. They don't have any hypoglicemic effect in absence of insulin, in spite of the precise mechanism of action has not been completely elucidated.

PPARγ is expressed in the adipose tissue and much less in liver, lung, colon and skeletal muscle. On the contrary, the hypoglicemic effect of TzDs seems due to the increase of glucose uptake in the skeletal muscle (80% of the glucose utilized by the body). The antagonizing activity of TzDs on the effects of TNF-α is able to determine an anti-inflammatory action. In the human macrophages TzDs are inhibiting the production of TNF-α as well as of other pro-inflammatory cytokines as IL-1β, IL-6 etc. In addiction through the interferences with the activity of the transcriptional factor NF-kB, AP 1 and STAT-1, the glitazones inhibit the expression of iNOS scavenger receptor A, gelatinase (MMP-9) and interleukin 8. Another important characteristics of the TzDs is the antiproliferative effect: the antitumoral action of these drugs has been observed in several tumours as liposarcoma breast, prostate, colon and thyroid cancer.

A large body of data seems to demonstrate important effects on endothelial cells. In rat models, troglitazone increases the NO levels stablizing mRNA of iNOS and decreases the expression of endothelis-1 and of inhibitor 1 of plasminogen activator which seems involved in atheroscherotic processes.

### Description of the figures

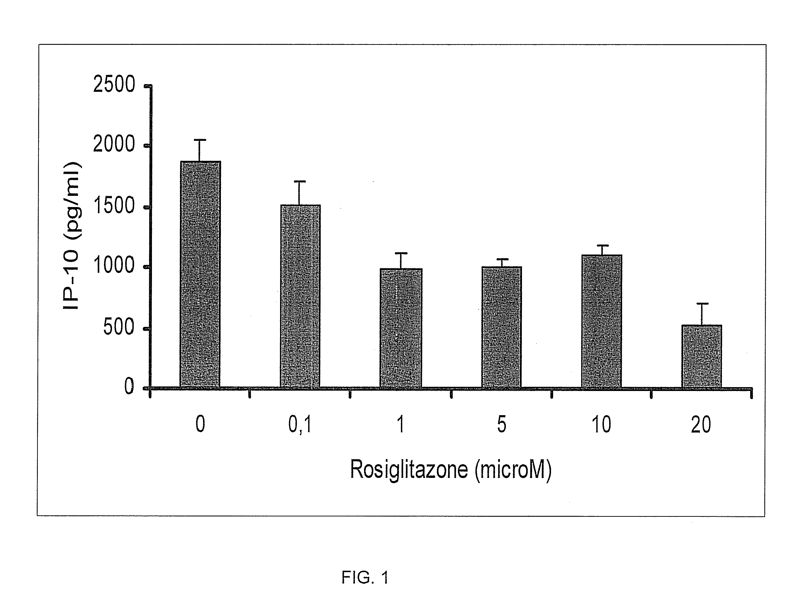

Fig. 1 shows the suppressive effects of Rosiglitazone on the IP-10 levels induced by interferon-γ in epithelial cells treated with increasing doses of Rosiglitazone.
In the figure the first column (0) indicates the IP-10 level after stimulation with INF-γ and TNF-α in the absence of TzDs.

### Detailed description of the invention

It has been surprisingly found that in addiction to above mentioned pharmacological actions TzDs have other therapeutic never described activities, which cannot be supposed on the basis of previous literature.

In fact, the suppressive effects of glitazones on the IP-10 levels induced by interferon gamma have been demonstrated by us in epithelial cells (in particular thyrociytes) (see figure 1).

Since we have demonstrated that in recently onset Graves disease high circulating levels of IP-10 are present and that the interferon-γ induced chemokines are produced in thyroid as well as in other endocrine glands not only by inflammatory infiltrates but also by epithelia, glitazones can be considered drugs able to block the evolution of recent onset autoimmune endocrine diseases.

The formulation to be used according the present invention are similar to those employed and commercialised usually as preparation and doses of active compound.

Also the daily doses can be comparable to those routinely used. For instance, formulation containing as active compound Rosiglitazone or Pioglitazone can be used as pills containing 15, 30, 45 mg of Piogliazone and 4 and 8 mg of Rosiglitazone.

## Claims

1. Use of thiazolidinediones for the preparation of pharmaceutical compositions for the treatment of endocrine autoimmune diseases.

2. Use according to claim 1 in which the endocrine disease is Graves disease.

3. Use according to claims 1 and 2 in which the above mentioned thiazolidinediones are Pioglitazone and Rosiglitazone.
